# EUROPÄISCHE PATENTSCHRIFT

(11) **EP 1 015 287 B1**
(45) Veröffentlichungstag und Bekanntmachung des Hinweises auf die Patenterteilung: **11.12.2002**
(21) Anmeldenummer: 98951457.5
(22) Anmeldetag: 23.09.1998
(51) Int. Cl.: G01J 1/04, B60S 1/08, B60H 1/00, B60Q 1/14

(54) **OPTOELEKTRONISCHE ÜBERWACHUNGSEINRICHTUNG FÜR EIN KRAFTFAHRZEUG**
OPTOELECTRONIC MONITORING DEVICE FOR A MOTOR VEHICLE
SYSTEME DE SURVEILLANCE OPTOELECTRONIQUE POUR AUTOMOBILE

(30) Priorität: 24.09.1997 DE 19742093
(43) Veröffentlichungstag der Anmeldung: 05.07.2000
(73) Patentinhaber: Leopold Kostal GmbH & Co. KG, 58507 Lüdenscheid (DE)
(72) Erfinder: BENDICKS, Norbert, D-58675 Hemer (DE); BLÄSING, Frank, D-59457 Werl (DE); KERKMANN, Detlef, D-58769 Nachrodt (DE); WEBER, Thomas, D-58513 Lüdenscheid (DE); BÖBEL, Ralf, D-44269 Dortmund (DE); DONNER, Harald, D-58540 Meinerzhagen (DE)
(74) Vertreter: Haverkamp, Jens
(86) Internationale Anmeldenummer: EP9806066
(87) Internationale Veröffentlichungsnummer: WO99015381

(56) Entgegenhaltungen:
- DE-A- 19 704 818
- GB-A- 2 311 602
- US-A- 4 288 819
- PATENT ABSTRACTS OF JAPAN vol. 010, no. 109 (M-472), 23. April 1986 & JP 60 240545 A (NIPPON DENSO KK), 29. November 1985

## Beschreibung

### Beschreibung für folgende Vertragsstaaten : DE, ES, FR, IT

Die Erfindung betrifft eine optoelektronische Überwachungseinrichtung für ein Kraftfahrzeug.

Eine derartige Überwachungseinrichtung ist beispielsweise aus der US-PS 4 867 561 bekannt. Diese Überwachungseinrichtung ist als Regensensor eingesetzt und besteht aus einem Abbildungssystem zum Abbilden von auf der Windschutzscheibe befindlichen Wassertropfen auf einem optoelektronischen Sensorarray, welches als CCD-Zeile ausgebildet ist. Der Regensensor umfaßt eine IR-lichtemittierende Einheit, deren Licht zum Detektieren von Wassertröpfchen auf der Windschutzscheibe zu dieser hingerichtet ist und wobei bei Vorhandensein von Wassertröpfchen auf der Windschutzscheibe eine Reflektion des emittierten Lichtes erfolgt. Das reflektierte Licht wird einer abbildenden konvexen Linse zugeführt, hinter der die CCD-Zeile als photoelektrisches Sensorarray angeordnet ist. In diesem Fall dient die Linse als Abbildungssystem zum Abbilden der von dem Sensorarray zu erfassenden optischen Information.

In einer Ausgestaltung dieses vorbekannten Regensensors ist vorgesehen, zwei IR-Lichtquellen zu verwenden, wobei die eine auf die äußere Oberfläche der Windschutzscheibe und die andere auf die innere Oberfläche der Windschutzscheibe abgestimmt ist. Dadurch ist eine Unterscheidung zwischen auf der Außenseite der Windschutzscheibe befindlichen Regentropfen und auf der Innenseite der Windschutzscheibe befindlichen Wassertröpfchen (Beschlag) möglich. Zu diesem Zweck sind eine bestimmte Anzahl nebeneinander liegender Wandlerelemente der CCD-Zeile zum Auswerten in Wandlerelementgruppen zusammengefaßt. Dabei ist vorgesehen, eine solche Gruppe der für das Detektieren von Regentropfen vorgesehenen Lichtquelle und eine weitere Gruppe der für das Detektieren eines Beschlages vorgesehenen Lichtquelle zuzuordnen.

Insgesamt stellt diese vorbekannte Vorrichtung einen Regen- bzw. Feuchtigkeitssensor dar, mit dem ausschließlich feststellbar ist, ob Wassertröpfchen auf der Außen- oder auf der Innenseite einer Windschutzscheibe vorhanden sind.

Aus der DE 197 04 818 A1 ist ein optischer Sensor bekannt, bei dem unterschiedlichen Überwachungsobjekten zugeordnete optische Überwachungssysteme ein photoelektrisches Sensorarray, etwa ein CCD-Array beaufschlagen. Diese optoelektronische Überwachungseinrichtung dient zum einen einer Kontrasterkennung der Kraftfahrzeugumgebung, beispielsweise zur Detektion von Tunnelein- und -ausfahrten. Das eingesetzte Abbildungssystem besteht aus einer optischen Sammellinse, die auf die Umgebung des Kraftfahrzeuges nach vorne gerichtet fokussiert ist. In einer weiteren Anwendung dieser Überwachungseinrichtung dient diese als Regensensor. Zu diesem Zweck wird in den Strahlengang des zuvor beschriebenen Abbildungssystems die Rückstreustrahlung von innenseitig die Windschutzscheibe beleuchteten Lichtstrahlen eingebracht, wobei die am Sensorarray empfangene Rückstreuung ein Maß für die Benetzung der Windschutzscheibe ist. Ausgangsseitig ist an das Sensorarray eine Auswerteeinheit zum Ansteuern von Aktoren in Abhängigkeit von dem jeweiligen Ergebnis einer Auswertung der objektbezogenen Informationen angeordnet.

Im Automotivebereich werden optische Sensoren zum Erfassen vielfältiger optischer Informationen verwendet. Beispielsweise finden derartige Sensorsysteme Anwendung bei der Erfassung des Sonnenstandes, bei der Fahrlichtsteuerung oder etwa bei einer Innenraumüberwachung. Sämtliche dieser Systeme verfügen über entsprechende photoelektrische Sensorarrays sowie über entsprechende Abbildungssysteme.

Ausgehend von diesem diskutiertem Stand der Technik liegt der Erfindung daher die Aufgabe zugrunde, eine optoelektronische Überwachungseinrichtung vorzuschlagen, mit der unterschiedliche optische Informationen verschiedener optischer Sensorsysteme erfaßbar und auf einem Sensorarray abbildbar sind.

Diese Aufgabe-wird erfindungsgemäß durch eine optoelektronische Überwachungseinrichtung für ein Kraftfahrzeug mit mehreren unterschiedlichen Überwachungsobjekten zugeordneten optischen Abbildungssystemen gelöst, die ausgangsseitig die photosensitive Oberfläche eines gemeinsamen photoelektrischen Sensorarrays, bestehend aus einer Vielzahl von einzelne Bildpunkte bildenden photoelektrischen Wandlerelementen in einer zweidimensionalen Anordnung, beaufschlagen, welche Wandlerelemente in Abhängigkeit von ihrer jeweiligen Lichtbeaufschlagung ein der Lichtstärke entsprechendes elektrisches Signal generieren, wobei der Ausgang eines jeden Abbildungssystems zum Abbilden der von diesem Abbildungssystem bereitgestellten optischen Information über einer diesem Abbildungssystem zugeordneten, aus einem oder mehreren Wandlerelementen bestehende Wandlerelementgruppe angeordnet ist, sowie mit einer durch die elektrischen Ausgangssignale des Sensorarrays beaufschlagten Auswerteeinheit zum Ansteuern von Aktoren in Abhängigkeit von dem Ergebnis einer Auswertung der Objekt bezogenen Informationen, welche Abbildungssysteme im Bereich ihrer Ausgänge zu einer gegenständlichen Einheit zusammengefaßt und auf einem dem Sensorarray zugeordneten Schaltungsträger fixiert sind.

Durch die erfindungsgemäße Überwachungseinrichtung, der mehrere Abbildungssysteme, zweckmäßigerweise von einem gemeinsamen, die Abbildungssysteme zusammenfassenden Halter getragen, zugeordnet sind, kann diese Überwachungseinrichtung unter Verwendung eines einzigen Sensorarrays für die unterschiedlichsten optischen Sensorsysteme eingesetzt werden. Als Abbildungssysteme kommen sowohl bildabbildende, etwa Linsen, oder lichtleitende Systeme in Frage. Da dem Ausgang jedes Abbildungssystems eine bestimmte Wandlerelementgruppe des Sensorarrays zugeordnet ist, ist eine eindeutige Zuordnung bestimmter Gruppensignale zu bestimmten Abbildungssystemen und somit zu bestimmten zu erfassenden optischen Informationen möglich. Dabei kann vorgesehen sein, daß zwischen den einzelnen Wandlerelementgruppen Wandlerelementspalten bzw. -zeilen vorgesehen sind, die keinem Abbildungssystem zugeordnet sind. Mit diesen Wandlerelementen ließe sich eine Belichtungssteuerung entsprechend dem Umgebungslicht oder eine Bestimmung des Gleichlichtanteiles durchführen.

Die einzelnen Abbildungssysteme sind im Bereich des photoelektrischen Sensorarrays zu einer gegenständlichen Einheit zusammengefaßt und auf dem Schaltungsträger des Sensorarrays fixiert. Somit ist das Sensorarray bezüglich der Ausgänge der Abbildungssysteme in einer fixierten Position gehalten, so daß auch bei Auftreten von Fahrzeugvibrationen von den Abbildungssystemen immer dieselben Wandlerelemente beaufschlagt werden.

Bei Bestückung eines in einem Kraftfahrzeug verwendeten photoelektrischen Sensorarrays mit einer Linse als eines der Abbildungssysteme ist dieses zum Erfassen bildabbildender Informationen geeignet. Ein solches System läßt sich daher beispielsweise als Innenraumüberwachungssystem oder auch zur Überwachung der Umgebung des Kraftfahrzeuges, etwa zum Signalempfang bei einer Abstandssensorik verwenden. Bei Verwendung dieses Sensorarrays zur Innenraumüberwachung ist es zweckmäßig, ein solches Sensorarray im Bereich des inneren Rückspiegels bzw. im Bereich einer Dachkonsole, etwa als Dachmodul nach hinten blickend anzuordnen. Die übrigen optischen Informationen werden dem Sensorarray mittels optischen Lichtleitern als weitere Abbildungssysteme zugeführt, wobei einzelne Fasern zur Übermittlung etwa von Lichtstärken oder Faserbündel zur Übermittlung von bildabbildenden Informationen eingesetzt sein können.

Ein solches photoelektrisches Sensorarray mit einer durch Zusammenfassung verschiedener Abbildungssysteme gebildeten Multifunktionsoptik kann auch an anderer Position in einem Kraftfahrzeug angeordnet sein. Die dem Sensorarray zugeführte optische Information kann bereits hinsichtlich der tatsächlich zu erfassenden Information gefiltert sein. Da das Sensorarray zur Aufnahme einer großen Frequenzbandbreite ausgestaltet sein kann, ist ein solches Sensorarray gleichzeitig für die unterschiedlichsten optischen Sensoriken geeignet, die sowohl IR-Licht, sichtbares Licht oder UV-Licht verwenden können.

In einer vorteilhaften Weiterbildung ist als Sensorarray ein solches vorgesehen, dessen einzelne Wandlerelemente frei adressierbar und auslesbar sind. Eine solche Ausgestaltung bringt insbesondere Vorteile hinsichtlich einer Ausrichtung der sensorarrayseitigen Ausgänge der Abbildungssysteme zur photosensitiven Oberfläche des Sensorarrays bei einer Montage der beiden Elemente sowie in Bezug auf eine Gewährleistung einer gleichbleibenden Qualität. Aufgrund der gleichartigen Ausbildung aller Wandlerelemente des Sensorarrays läßt sich bei dieser Ausgestaltung die räumlich Lage einer Wandlerelementgruppe softwaremäßig definieren. Es ist dann eine softwaregesteuerte Justage der Wandlerelementgruppen bezüglich der jeweiligen Abbildungssysteme möglich. Dadurch lassen sich Montageungenauigkeiten hinsichtlich der Anordnung der Abbildungssysteme zum Sensorarray ausgleichen; ebenfalls sind die einzelnen Wandlerelementgruppen an Veränderungen, die sich infolge eines langjährigen Betriebes oder infolge anderer Ursachen, die zu einer Abweichung der für ein Abbildungssystem definierten Wandlerelementgruppe führen, softwaremäßig adaptierbar.

Weitere Vorteile der Erfindung sowie Weiterbildungen sind Bestandteil der abhängigen Ansprüche sowie der nachfolgenden Beschreibung eines Ausführungsbeispieles. Es zeigen:
- **Fig. 1:**: einen schematisierten Schnitt durch ein photoelektrisches Sensorarray mit mehreren Abbildungssystemen einer optoelektronischen Überwachungseinrichtung für ein Kraftfahrzeug,
- **Fig. 2:**: eine vergrößerte Draufsicht auf das Sensorarray der Figur 1 und
- **Fig. 3**: eine schematische Ansicht durch ein weiteres photoelektrisches Sensorarrays mit mehreren Abbildungssystemen einer optoelektronischen Überwachungseinrichtung für ein Kraftfahrzeug.

Auf einer Leiterplatte 1 ist ein photoelektrischer Sensorarray 2 mit seinen Kontaktfüßen 3 entsprechende Leiterbahnen elektrisch kontaktierend befestigt. Bei dem photoelektrischen Sensorarray 2, handelt es sich um einen quadratischen Kamerasensorchip, dessen photosensitive Oberfläche durch eine Vielzahl von einzelnen Wandlerelementen (Pixeln) gebildet wird. Bei dem dargestellten photoelektrischen Sensorarray sind quadratische Pixel vorgesehen, so daß das Sensorarray 2 eine der Spaltenpixelzahl entsprechende Zeilenpixelzahl aufweist. Die einzelnen Pixel des Kamerasensorchips 2 sind frei adressierbar und auslesbar.

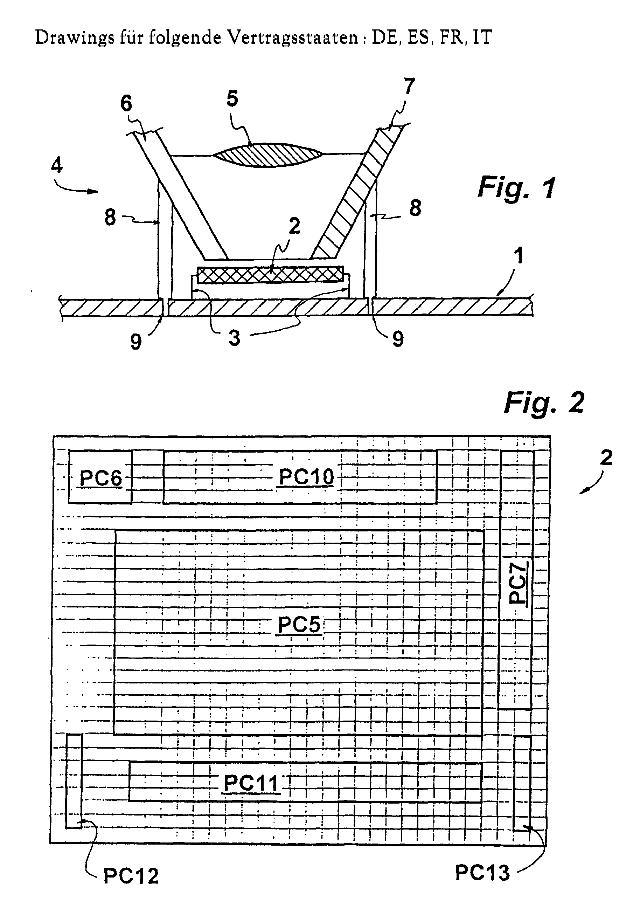

Oberhalb des Sensorarrays 2 ist eine in einem nicht näher dargestellten Halter gehaltene Multifunktionsoptik 4 angeordnet. Die Multifunktionsoptik 4 stellt einen Optikkörper dar, in dem mehrere Abbildungssysteme 5, 6, 7 zusammengefaßt sind. Die Abbildungssysteme 5, 6, 7 führen der photosensitven Oberfläche des Sensorarrays 2 die für die Erfassung bestimmter Systeme benötigte optische Information zu. Bei dem Abbildungssystem 5 handelt es sich um eine abbildende Optik, nämlich um eine konvexe Linse; bei den Abbildungssystemen 6, 7 handelt es sich jeweils um optische Lichtleiter, die die benötigte optische Information von dem eigentlichen Ort der Erfassung dem Sensorarray 2 zuführen. Das Abbildungssystem 5 dient bei dem in Figur 1 dargestellten Ausführungsbeispiel zur Innenraumüberwachung eines Kraftfahrzeuges. Die Abbildungssysteme 6, 7 dienen der Darstellung einer Regensensorik bzw. einer Sonnenstandssensorik.

Die Multifunktionsoptik 4 bzw. der Halter des Optikkörpers ist über Füße 8 abgestützt auf der Leiterplatte 1 in Auffangbohrungen 9 gehalten und befestigt.

Aus der Darstellung der Figur 1 wird deutlich, daß bei der Montage sowohl des Kamerasensorchips 2 als auch der Multifunktionsoptik 4 Toleranzungenauigkeiten hinsichtlich der Zuordnung des Ausgangs eines Abbildungssystems 5, 6 oder 7 zu einem bestimmten diesen Ausgängen zugeordneten Pixelcluster auftreten können. Eine exakte, an die tatsächlichen Ausgänge der Abbildungssysteme 5, 6, 7 nach einer Montage der Einheiten 2, 4 auf einer Leiterplatte 1 adaptierte Justage der Einheiten 2, 4 zueinander erfolgt durch eine softwaregesteuerte Pixelclusterung, so daß genau diejenigen Pixel des Sensorarrays 2 dem jeweiligen Ausgang eines Abbildungssystemes 5, 6, 7 zugeordnet sind, die auch tatsächlich von dem Ausgang eines solchen Abbildungssystemes 5, 6, 7 lichtbeaufschlagt sind. Zu diesem Zweck sind die einzelnen Pixel frei adressierbar. Entsprechend lassen sich langsamere Veränderungen infolge des Betriebes dieses Systems ausgleichen.

Aus der in Figur 2 gezeigten Draufsicht auf die photosensitive Seite des Kamerasensorchips 2 wird die Clusterung der Pixel deutlich. Dem Abbildungssystem 5 ist das Pixelcluster PC 5, dem Abbildungssystem 6 das Pixelcluster PC 6 und dem Abbildungssystem 7 das Pixelcluster PC 7 zugeordnet. Neben den Abbildungssystemen 5, 6, 7 sind in der Multifunktionsoptik 4 weitere, in Figur 1 der Übersicht halber nicht dargestellte Abbildungssysteme enthalten, deren Pixelcluster in Figur 2 mit PC 10, PC 11, PC 12 und PC 13 bezeichnet sind. Die einzelnen Pixelcluster PC 5, PC 6, PC 7, PC 10, PC 11, PC 12, PC 13 grenzen nicht unmittelbar aneinander, sondern sind durch einzelne Pixelreihen bzw. -spalten voneinander getrennt. Diese, dem Ausgang eines Abbildungssystems nicht unmittelbar zugeordneten Pixel können entweder ungenutzt und somit softwaremäßig nicht angesteuert sein, oder aber diese können etwa der Belichtungssteuerung oder zur Bestimmung des Gleichlichtanteiles verwendet werden.

In einem weiteren, nicht dargestellten Ausführungsbeispiel sind die Ausgänge der verwendeten Abbildungssysteme so angeordnet, daß diese quasi unmittelbar aneinandergrenzen, wobei diese sich jedoch gegenseitig nicht beeinflussen.

Durch die freie Auslesbarkeit der einzelnen Pixelcluster PC 5, PC 6, PC 7, PC 10, PC 11, PC 12, PC 13 können die Signale getrennten Auswerteverfahren zugeführt werden. Entsprechend ist es möglich, den einzelnen Pixeldustem PC 5 bis PC 13 unterschiedliche Bedeutungen bzw. Prioritäten zuzuordnen, anhand deren sich die Rangfolge ihres Auslesens orientiert.

Der Kamerasensorchip 2 ist an eine in den Figuren nicht dargestellte Prozessoreinheit angeschlossen, durch die die einzelnen Pixelcluster PC 5 bis PC 13 des Kamerasensorchips 2 entsprechend einem vorgegebenen Algorithmus ausgelesen werden.

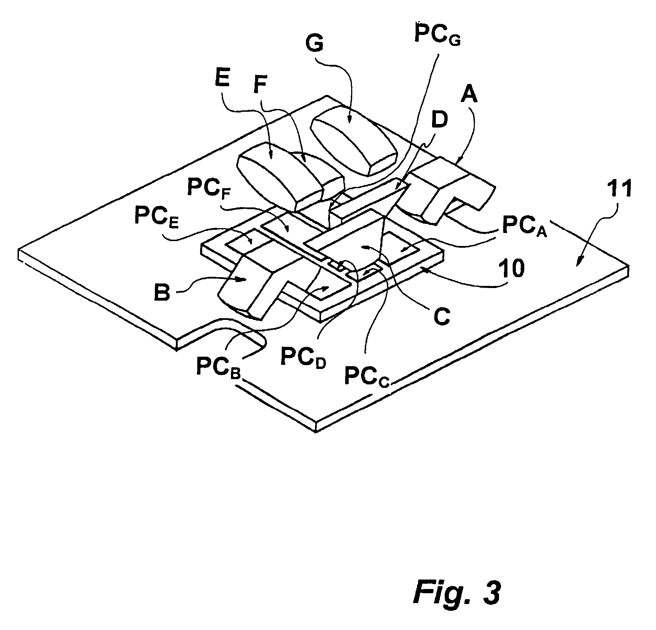

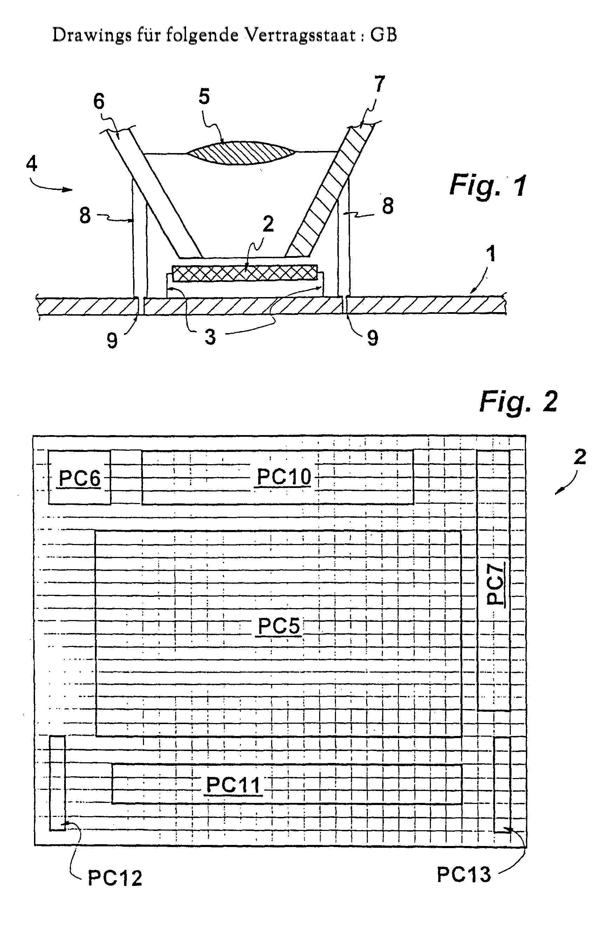

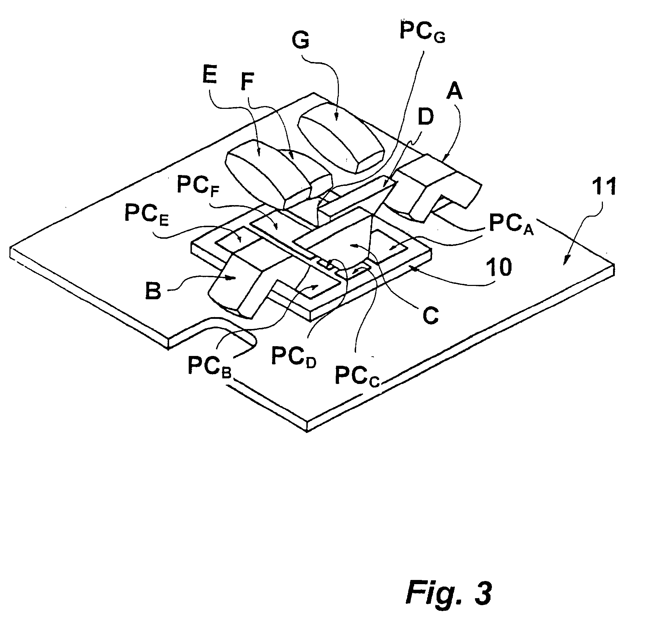

Nach Art einer perspektivischen Ansicht ist in Figur 3 schematisiert die Aufnahmeeinheit einer weiteren optoelektronischen Überwachungseinrichtung für ein Kraftfahrzeug dargestellt. Die Aufnahmeeinheit umfaßt wiederum einen Flächenkamerachip 10, der auf einer Trägerplatte 11 montiert ist. In einem nicht näher dargestellten Halter sind mehrere Abbildungssysteme A - G zusammengefaßt und von diesem in ihrer in Figur 3 dargestellten Position bezüglich der photosensitiven Oberfläche des Flächenkamerachips 10 gehalten. Die Abbildungssysteme A - G führen optische Informationen von unterschiedlichen Überwachungsobjekten dem Flächenkamerachip 10 zu. Jedem Abbildungssystem A - G ist ein diskretes Pixelcluster PC_{A} - PC_{G} zugeordnet. Bei den Abbildungssystemen E - G handelt es sich um bildabbildende Abbildungssysteme, wobei die in Figur 3 dargestellten Optiken nach Art konvexer Linsen ausgebildet sind. Die übrigen Abbildungssysteme A - D dienen der Übermittlung von lichtabbildenden Informationen, wie beispielsweise Helligkeitsunterschieden.

Es ist besonders vorteilhaft, die dargestellten Abbildungssysteme A - G aus einem Kunststoff herzustellen, wobei die dargestellten Optikkörper durch Stege miteinander verbunden sind. Die Stege stellen dann den gemeinsamen Halter der Abbildungssysteme A - G dar, wobei diese sich über Füße auf der Trägerplatte 11 abstützen. Dabei kann vorgesehen sein, daß diese zusammengehaltenen Abbildungssysteme durch einen rastenden Steckvorgang mit der Trägerplatte 11 verbindbar sind.

In einer Weiterführung ist vorgesehen, daß einzelne Pixelcluster nicht einheitlich, sondern in Blöcke segmentiert blockweise ausgelesen werden, wie dies etwa aus der US-PS 4 867 561 bekannt ist.

Aus der Darstellung der Erfindung wird deutlich, daß das beschriebene photoelektrische Sensorarray mit seiner Multifunktionsoptik für zahlreiche Anwendungsfälle geeignet ist, von denen hier beispielsweise die Anwendung im Automotivebereich dargelegt ist. Die Möglichkeit der zentralen Anordnung eines solchen Systems wirkt sich auch günstig auf die Variabilität eines solchen Systems aus, beispielsweise wenn eine Multifunktionsoptik freie Aufnahmen zur nachträglichen Installation zusätzlicher Abbildungssysteme aufweist. Durch einfaches Umprogrammieren der Pixelcluster bzw. durch zusätzliches Aktivieren bestimmter Pixelcluster läßt sich der Kamerasensorchip 2 entsprechend einrichten.

### Zusammenstellung der Bezugszeichen

- 1: Leiterplatte
- 2: photoelektrisches Sensorarray, Kamerasensorchip
- 3: Kontaktfuß
- 4: Multifunktionsoptik
- 5: Abbildungssystem
- 6: Abbildungssystem
- 7: Abbildungssystem
- 8: Fuß
- 9: Auffangbohrung
- 10: Flächenkamerachip
- 11: Trägerplatte

- PC 5 - PC 13: Pixelcluster
- A - G: Abbildungssystem
- PC_{A}-PC_{G}: Pixelcluster

### Beschreibung für folgenden Vertragsstaat : GB

Die Erfindung betrifft eine optoelektronische Überwachungseinrichtung für ein Kraftfahrzeug.

Eine derartige Überwachungseinrichtung ist beispielsweise aus der US-PS 4 867 561 bekannt. Diese Überwachungseinrichtung ist als Regensensor eingesetzt und besteht aus einem Abbildungssystem zum Abbilden von auf der Windschutzscheibe befindlichen Wassertropfen auf einem optoelektronischen Sensorarray, welches als CCD-Zeile ausgebildet ist. Der Regensensor umfaßt eine IR-lichtemittierende Einheit, deren Licht zum Detektieren von Wassertröpfchen auf der Windschutzscheibe zu dieser hingerichtet ist und wobei bei Vorhandensein von Wassertröpfchen auf der Windschutzscheibe eine Reflektion des emittierten Lichtes erfolgt. Das reflektierte Licht wird einer abbildenden konvexen Linse zugeführt, hinter der die CCD-Zeile als photoelektrisches Sensorarray angeordnet ist. In diesem Fall dient die Linse als Abbildungssystem zum Abbilden der von dem Sensorarray zu erfassenden optischen Information.

In einer Ausgestaltung dieses vorbekannten Regensensors ist vorgesehen, zwei IR-Lichtquellen zu verwenden, wobei die eine auf die äußere Oberfläche der Windschutzscheibe und die andere auf die innere Oberfläche der Windschutzscheibe abgestimmt ist. Dadurch ist eine Unterscheidung zwischen auf der Außenseite der Windschutzscheibe befindlichen Regentropfen und auf der Innenseite der Windschutzscheibe befindlichen Wassertröpfchen (Beschlag) möglich. Zu diesem Zweck sind eine bestimmte Anzahl nebeneinander liegender Wandlerelemente der CCD-Zeile zum Auswerten in Wandlerelementgruppen zusammengefaßt. Dabei ist vorgesehen, eine solche Gruppe der für das Detektieren von Regentropfen vorgesehenen Lichtquelle und eine weitere Gruppe der für das Detektieren eines Beschlages vorgesehenen Lichtquelle zuzuordnen.

Insgesamt stellt diese vorbekannte Vorrichtung einen Regen- bzw. Feuchtigkeitssensor dar, mit dem ausschließlich feststellbar ist, ob Wassertröpfchen auf der Außen- oder auf der Innenseite einer Windschutzscheibe vorhanden sind.

Aus der DE 197 04 818 A1 ist ein optischer Sensor bekannt, bei dem unterschiedlichen Überwachungsobjekten zugeordnete optische Überwachungssysteme ein photoelektrisches Sensorarray, etwa ein CCD-Array beaufschlagen. Diese optoelektronische Überwachungseinrichtung dient zum einen einer Kontrasterkennung der Kraftfahrzeugumgebung, beispielsweise zur Detektion von Tunnelein- und -ausfahrten. Das eingesetzte Abbildungssystem besteht aus einer optischen Sammellinse, die auf die Umgebung des Kraftfahrzeuges nach vorne gerichtet fokussiert ist. In einer weiteren Anwendung dieser Überwachungseinrichtung dient diese als Regensensor. Zu diesem Zweck wird in den Strahlengang des zuvor beschriebenen Abbildungssystems die Rückstreustrahlung von innenseitig die Windschutzscheibe beleuchteten Lichtstrahlen eingebracht, wobei die am Sensorarray empfangene Rückstreuung ein Maß für die Benetzung der Windschutzscheibe ist. Ausgangsseitig ist an das Sensorarray eine Auswerteeinheit zum Ansteuern von Aktoren in Abhängigkeit von dem jeweiligen Ergebnis einer Auswertung der objektbezogenen Informationen angeordnet.

Im Automotivebereich werden optische Sensoren zum Erfassen vielfältiger optischer Informationen verwendet. Beispielsweise finden derartige Sensorsysteme Anwendung bei der Erfassung des Sonnenstandes, bei der Fahrlichtsteuerung oder etwa bei einer Innenraumüberwachung. Sämtliche dieser Systeme verfügen über entsprechende photoelektrische Sensorarrays sowie über entsprechende Abbildungssysteme.

Ausgehend von diesem diskutiertem Stand der Technik liegt der Erfindung daher die Aufgabe zugrunde, eine optoelektronische Überwachungseinrichtung vorzuschlagen, mit der unterschiedliche optische Informationen verschiedener optischer Sensorsysteme erfaßbar und auf einem Sensorarray abbildbar sind.

Diese Aufgabe wird erfindungsgemäß durch eine optoelektronische Überwachungseinrichtung für ein Kraftfahrzeug mit mehreren unterschiedlichen Überwachungsobjekten zugeordneten optischen Abbildungssystemen gelöst, die ausgangsseitig die photosensitive Oberfläche eines gemeinsamen photoelektrischen Sensorarrays, bestehend aus einer Vielzahl von einzelne Bildpunkte bildenden photoelektrischen Wandlerelementen in einer zweidimensionalen Anordnung, beaufschlagen, welche Wandlerelemente in Abhängigkeit von ihrer jeweiligen Lichtbeaufschlagung ein der Lichtstärke entsprechendes elektrisches Signal generieren, wobei der Ausgang eines jeden Abbildungssystems zum Abbilden der von diesem Abbildungssystem bereitgestellten optischen Information über einer diesem Abbildungssystem zugeordneten, aus einem oder mehreren Wandlerelementen bestehende Wandlerelementgruppe angeordnet ist, sowie mit einer durch die elektrischen Ausgangssignale des Sensorarrays beaufschlagten Auswerteeinheit zum Ansteuern von Aktoren in Abhängigkeit von dem Ergebnis einer Auswertung der Objekt bezogenen Informationen, welche Abbildungssysteme im Bereich ihrer Ausgänge zu einer gegenständlichen Einheit zusammengefaßt und auf einem dem Sensorarray zugeordneten Schaltungsträger fixiert sind.

Durch die erfindungsgemäße Überwachungseinrichtung, der mehrere Abbildungssysteme, zweckmäßigerweise von einem gemeinsamen, die Abbildungssysteme zusammenfassenden Halter getragen, zugeordnet sind, kann diese Überwachungseinrichtung unter Verwendung eines einzigen Sensorarrays für die unterschiedlichsten optischen Sensorsysteme eingesetzt werden. Als Abbildungssysteme kommen sowohl bildabbildende, etwa Linsen, oder lichtleitende Systeme in Frage. Da dem Ausgang jedes Abbildungssystems eine bestimmte Wandlerelementgruppe des Sensorarrays zugeordnet ist, ist eine eindeutige Zuordnung bestimmter Gruppensignale zu bestimmten Abbildungssystemen und somit zu bestimmten zu erfassenden optischen Informationen möglich. Dabei kann vorgesehen sein, daß zwischen den einzelnen Wandlerelementgruppen Wandlerelementspalten bzw. -zeilen vorgesehen sind, die keinem Abbildungssystem zugeordnet sind. Mit diesen Wandlerelementen ließe sich eine Belichtungssteuerung entsprechend dem Umgebungslicht oder eine Bestimmung des Gleichlichtanteiles durchführen.

Die einzelnen Abbildungssysteme sind im Bereich des photoelektrischen Sensorarrays zu einer gegenständlichen Einheit zusammengefaßt und auf dem Schaltungsträger des Sensorarrays fixiert. Somit ist das Sensorarray bezüglich der Ausgänge der Abbildungssysteme in einer fixierten Position gehalten, so daß auch bei Auftreten von Fahrzeugvibrationen von den Abbildungssystemen immer dieselben Wandlerelemente beaufschlagt werden.

Bei Bestückung eines in einem Kraftfahrzeug verwendeten photoelektrischen Sensorarrays mit einer Linse als eines der Abbildungssysteme ist dieses zum Erfassen bildabbildender Informationen geeignet. Ein solches System läßt sich daher beispielsweise als Innenraumüberwachungssystem oder auch zur Überwachung der Umgebung des Kraftfahrzeuges, etwa zum Signalempfang bei einer Abstandssensorik verwenden. Bei Verwendung dieses Sensorarrays zur Innenraumüberwachung ist es zweckmäßig, ein solches Sensorarray im Bereich des inneren Rückspiegels bzw. im Bereich einer Dachkonsole, etwa als Dachmodul nach hinten blickend anzuordnen. Die übrigen optischen Informationen werden dem Sensorarray mittels optischen Lichtleitern als weitere Abbildungssysteme zugeführt, wobei einzelne Fasern zur Übermittlung etwa von Lichtstärken oder Faserbündel zur Übermittlung von bildabbildenden Informationen eingesetzt sein können.

Ein solches photoelektrisches Sensorarray mit einer durch Zusammenfassung verschiedener Abbildungssysteme gebildeten Multifunktionsoptik kann auch an anderer Position in einem Kraftfahrzeug angeordnet sein. Die dem Sensorarray zugeführte optische Information kann bereits hinsichtlich der tatsächlich zu erfassenden Information gefiltert sein. Da das Sensorarray zur Aufnahme einer großen Frequenzbandbreite ausgestaltet sein kann, ist ein solches Sensorarray gleichzeitig für die unterschiedlichsten optischen Sensoriken geeignet, die sowohl IR-Licht, sichtbares Licht oder UV-Licht verwenden können.

In einer vorteilhaften Weiterbildung ist als Sensorarray ein solches vorgesehen, dessen einzelne Wandlerelemente frei adressierbar und auslesbar sind. Eine solche Ausgestaltung bringt insbesondere Vorteile hinsichtlich einer Ausrichtung der sensorarrayseitigen Ausgänge der Abbildungssysteme zur photosensitiven Oberfläche des Sensorarrays bei einer Montage der beiden Elemente sowie in Bezug auf eine Gewährleistung einer gleichbleibenden Qualität. Aufgrund der gleichartigen Ausbildung aller Wandlerelemente des Sensorarrays läßt sich bei dieser Ausgestaltung die räumlich Lage einer Wandlerelementgruppe softwaremäßig definieren. Es ist dann eine softwaregesteuerte Justage der Wandlerelementgruppen bezüglich der jeweiligen Abbildungssysteme möglich. Dadurch lassen sich Montageungenauigkeiten hinsichtlich der Anordnung der Abbildungssysteme zum Sensorarray ausgleichen; ebenfalls sind die einzelnen Wandlerelementgruppen an Veränderungen, die sich infolge eines langjährigen Betriebes oder infolge anderer Ursachen, die zu einer Abweichung der für ein Abbildungssystem definierten Wandlerelementgruppe führen, softwaremäßig adaptierbar.

Weitere Vorteile der Erfindung sowie Weiterbildungen sind Bestandteil der abhängigen Ansprüche sowie der nachfolgenden Beschreibung eines Ausführungsbeispieles. Es zeigen:
- **Fig. 1:**: einen schematisierten Schnitt durch ein photoelektrisches Sensorarray mit mehreren Abbildungssystemen einer optoelektronischen Überwachungseinrichtung für ein Kraftfahrzeug,
- **Fig. 2:**: eine vergrößerte Draufsicht auf das Sensorarray der Figur 1 und
- **Fig. 3**: eine schematische Ansicht durch ein weiteres photoelektrisches Sensorarrays mit mehreren Abbildungssystemen einer optoelektronischen Überwachungseinrichtung für ein Kraftfahrzeug.

Auf einer Leiterplatte 1 ist ein photoelektrischer Sensorarray 2 mit seinen Kontaktfüßen 3 entsprechende Leiterbahnen elektrisch kontaktierend befestigt. Bei dem photoelektrischen Sensorarray 2, handelt es sich um einen quadratischen Kamerasensorchip, dessen photosensitive Oberfläche durch eine Vielzahl von einzelnen Wandlerelementen (Pixeln) gebildet wird. Bei dem dargestellten photoelektrischen Sensorarray sind quadratische Pixel vorgesehen, so daß das Sensorarray 2 eine der Spaltenpixelzahl entsprechende Zeilenpixelzahl aufweist. Die einzelnen Pixel des Kamerasensorchips 2 sind frei adressierbar und auslesbar.

Oberhalb des Sensorarrays 2 ist eine in einem nicht näher dargestellten Halter gehaltene Multifunktionsoptik 4 angeordnet. Die Multifunktionsoptik 4 stellt einen Optikkörper dar, in dem mehrere Abbildungssysteme 5, 6, 7 zusammengefaßt sind. Die Abbildungssysteme 5, 6, 7 führen der photosensitven Oberfläche des Sensorarrays 2 die für die Erfassung bestimmter Systeme benötigte optische Information zu. Bei dem Abbildungssystem 5 handelt es sich um eine abbildende Optik, nämlich um eine konvexe Linse; bei den Abbildungssystemen 6, 7 handelt es sich jeweils um optische Lichtleiter, die die benötigte optische Information von dem eigentlichen Ort der Erfassung dem Sensorarray 2 zuführen. Das Abbildungssystem 5 dient bei dem in Figur 1 dargestellten Ausführungsbeispiel zur Innenraumüberwachung eines Kraftfahrzeuges. Die Abbildungssysteme 6, 7 dienen der Darstellung einer Regensensorik bzw. einer Sonnenstandssensorik.

Die Multifunktionsoptik 4 bzw. der Halter des Optikkörpers ist über Füße 8 abgestützt auf der Leiterplatte 1 in Auffangbohrungen 9 gehalten und befestigt.

Aus der Darstellung der Figur 1 wird deutlich, daß bei der Montage sowohl des Kamerasensorchips 2 als auch der Multifunktionsoptik 4 Toleranzungenauigkeiten hinsichtlich der Zuordnung des Ausgangs eines Abbildungssystems 5, 6 oder 7 zu einem bestimmten diesen Ausgängen zugeordneten Pixelcluster auftreten können. Eine exakte, an die tatsächlichen Ausgänge der Abbildungssysteme 5, 6, 7 nach einer Montage der Einheiten 2, 4 auf einer Leiterplatte 1 adaptierte Justage der Einheiten 2, 4 zueinander erfolgt durch eine softwaregesteuerte Pixelclusterung, so daß genau diejenigen Pixel des Sensorarrays 2 dem jeweiligen Ausgang eines Abbildungssystemes 5, 6, 7 zugeordnet sind, die auch tatsächlich von dem Ausgang eines solchen Abbildungssystemes 5, 6, 7 lichtbeaufschlagt sind. Zu diesem Zweck sind die einzelnen Pixel frei adressierbar. Entsprechend lassen sich langsamere Veränderungen infolge des Betriebes dieses Systems ausgleichen.

Aus der in Figur 2 gezeigten Draufsicht auf die photosensitive Seite des Kamerasensorchips 2 wird die Clusterung der Pixel deutlich. Dem Abbildungssystem 5 ist das Pixelcluster PC 5, dem Abbildungssystem 6 das Pixelcluster PC 6 und dem Abbildungssystem 7 das Pixelcluster PC 7 zugeordnet. Neben den Abbildungssystemen 5, 6, 7 sind in der Multifunktionsoptik 4 weitere, in Figur 1 der Übersicht halber nicht dargestellte Abbildungssysteme enthalten, deren Pixelcluster in Figur 2 mit PC 10, PC 11, PC 12 und PC 13 bezeichnet sind. Die einzelnen Pixelcluster PC 5, PC 6, PC 7, PC 10, PC 11, PC 12, PC 13 grenzen nicht unmittelbar aneinander, sondern sind durch einzelne Pixelreihen bzw. -spalten voneinander getrennt. Diese, dem Ausgang eines Abbildungssystems nicht unmittelbar zugeordneten Pixel können entweder ungenutzt und somit softwaremäßig nicht angesteuert sein, oder aber diese können etwa der Belichtungssteuerung oder zur Bestimmung des Gleichlichtanteiles verwendet werden.

In einem weiteren, nicht dargestellten Ausführungsbeispiel sind die Ausgänge der verwendeten Abbildungssysteme so angeordnet, daß diese quasi unmittelbar aneinandergrenzen, wobei diese sich jedoch gegenseitig nicht beeinflussen.

Durch die freie Auslesbarkeit der einzelnen Pixelcluster PC 5, PC 6, PC 7, PC 10, PC 11, PC 12, PC 13 können die Signale getrennten Auswerteverfahren zugeführt werden. Entsprechend ist es möglich, den einzelnen Pixelclustern PC 5 bis PC 13 unterschiedliche Bedeutungen bzw. Prioritäten zuzuordnen, anhand deren sich die Rangfolge ihres Auslesens orientiert.

Der Kamerasensorchip 2 ist an eine in den Figuren nicht dargestellte Prozessoreinheit angeschlossen, durch die die einzelnen Pixelcluster PC 5 bis PC 13 des Kamerasensorchips 2 entsprechend einem vorgegebenen Algorithmus ausgelesen werden.

Nach Art einer perspektivischen Ansicht ist in Figur 3 schematisiert die Aufnahmeeinheit einer weiteren optoelektronischen Überwachungseinrichtung für ein Kraftfahrzeug dargestellt. Die Aufnahmeeinheit umfaßt wiederum einen Flächenkamerachip 10, der auf einer Trägerplatte 11 montiert ist. In einem nicht näher dargestellten Halter sind mehrere Abbildungssysteme A - G zusammengefaßt und von diesem in ihrer in Figur 3 dargestellten Position bezüglich der photosensitiven Oberfläche des Flächenkamerachips 10 gehalten. Die Abbildungssysteme A - G führen optische Informationen von unterschiedlichen Überwachungsobjekten dem Flächenkamerachip 10 zu. Jedem Abbildungssystem A - G ist ein diskretes Pixelcluster PC_{A} - PC_{G} zugeordnet. Bei den Abbildungssystemen E - G handelt es sich um bildabbildende Abbildungssysteme, wobei die in Figur 3 dargestellten Optiken nach Art konvexer Linsen ausgebildet sind. Die übrigen Abbildungssysteme A - D dienen der Übermittlung von lichtabbildenden Informationen, wie beispielsweise Helligkeitsunterschieden.

Es ist besonders vorteilhaft, die dargestellten Abbildungssysteme A - G aus einem Kunststoff herzustellen, wobei die dargestellten Optikkörper durch Stege miteinander verbunden sind. Die Stege stellen dann den gemeinsamen Halter der Abbildungssysteme A - G dar, wobei diese sich über Füße auf der Trägerplatte 11 abstützen. Dabei kann vorgesehen sein, daß diese zusammengehaltenen Abbildungssysteme durch einen rastenden Steckvorgang mit der Trägerplatte 11 verbindbar sind.

In einer Weiterführung ist vorgesehen, daß einzelne Pixelcluster nicht einheitlich, sondern in Blöcke segmentiert blockweise ausgelesen werden, wie dies etwa aus der US-PS 4 867 561 bekannt ist.

Aus der Darstellung der Erfindung wird deutlich, daß das beschriebene photoelektrische Sensorarray mit seiner Multifunktionsoptik für zahlreiche Anwendungsfälle geeignet ist, von denen hier beispielsweise die Anwendung im Automotivebereich dargelegt ist. Die Möglichkeit der zentralen Anordnung eines solchen Systems wirkt sich auch günstig auf die Variabilität eines solchen Systems aus, beispielsweise wenn eine Multifunktionsoptik freie Aufnahmen zur nachträglichen Installation zusätzlicher Abbildungssysteme aufweist. Durch einfaches Umprogrammieren der Pixelcluster bzw. durch zusätzliches Aktivieren bestimmter Pixelcluster läßt sich der Kamerasensorchip 2 entsprechend einrichten.

### Zusammenstellung der Bezugszeichen

- 1: Leiterplatte
- 2: photoelektrisches Sensorarray, Kamerasensorchip
- 3: Kontaktfuß
- 4: Multifunktionsoptik
- 5: Abbildungssystem
- 6: Abbildungssystem
- 7: Abbildungssystem
- 8: Fuß
- 9: Auffangbohrung
- 10: Flächenkamerachip
- 11: Trägerplatte

- PC 5 - PC 13: Pixelcluster
- A - G: Abbildungssystem
- PC_{A}-PC_{G}: Pixelcluster

## Patentansprüche (Patentansprüche für folgende(n) Vertragsstaat(en): DE, ES, FR, IT)

1. Optoelektronische Überwachungseinrichtung für ein Kraftfahrzeug mit mehreren unterschiedlichen Überwachungsobjekten zugeordneten optischen Abbildungssystemen (5 - 7; A - G), die ausgangsseitig die photosensitive Oberfläche eines gemeinsamen photoelektrischen Sensorarrays (2, 10), bestehend aus einer Vielzahl von einzelne Bildpunkte bildenden photoelektrischen Wandlerelementen in einer zweidimensionalen Anordnung, beaufschlagen, welche Wandlerelemente in Abhängigkeit von ihrer jeweiligen Lichtbeaufschlagung ein der Lichtstärke entsprechendes elektrisches Signal generieren, wobei der Ausgang eines jeden Abbildungssystems (5 - 7; A - G) zum Abbilden der von diesem Abbildungssystem bereitgestellten optischen Information über einer diesem Abbildungssystem (5 - 7; A - G) zugeordneten, aus einem oder mehreren Wandlerelementen bestehende Wandlerelementgruppe (PC 5 - PC 13; PC_{A} - PC_{G}) angeordnet ist, sowie mit einer durch die elektrischen Ausgangssignale des Sensorarrays (2, 10) beaufschlagten Auswerteeinheit zum Ansteuern von Aktoren in Abhängigkeit von dem Ergebnis einer Auswertung der Objekt bezogenen Informationen, welche Abbildungssysteme im Bereich ihrer Ausgänge zu einer gegenständlichen Einheit zusammengefaßt und auf einem dem Sensorarray (2, 10) zugeordneten Schaltungsträger fixiert sind.

2. Einrichtung nach Anspruch 1, bei der die einzelnen Wandlerelemente des Sensorarrays (2, 10) frei adressierbar und auslesbar sind.

3. Einrichtung nach Anspruch 1 oder 2, bei der sich die in jeder Wandlerelementgruppe (PC 5 - PC 13; PC_{A} - PC_{G}) zusammenfaßten Wandlerelemente von denjenigen der in den übrigen übrigen Wandlerelementgruppen (PC 5 - PC 13; PC_{A} - PC_{G}) zusammengefaßten Wandlerelemente unterscheiden.

4. Einrichtung nach einem der Ansprüche 1 bis 3, bei der zumindest eines der Abbildungssysteme (5 - 7; A - G) einen bildabbildenden Optikkörper, etwa eine Linse (5, E - F) aufweist und andere als optische Lichtleiter (6, 7, A - D) ausgebildet sind.

5. Einrichtung nach Anspruch 4, bei welcher der Optikkörper (5) zur Abbildung des Innenraums zur Durchführung einer Innenraumüberwachung des Kraftfahrzeugs und die Lichtleiter (6, 7) zur Durchführung etwa einer Regensensorik, einer Sonnenstandssensorik oder einer Fahrlichtsteuerung vorgesehen sind.

## Patentansprüche (Patentansprüche für folgende(n) Vertragsstaat(en): GB)

1. Optoelektronische Überwachungseinrichtung für ein Kraftfahrzeug mit mehreren unterschiedlichen Überwachungsobjekten zugeordneten optischen Abbildungssystemen (5 - 7; A - G), die ausgangsseitig die photosensitive Oberfläche eines gemeinsamen photoelektrischen Sensorarrays (2, 10), bestehend aus einer Vielzahl von einzelne Bildpunkte bildenden, photoelektrischen, frei adressier- und auslesbaren Wandlerelementen in einer zweidimensionalen Anordnung, beaufschlagen, welche Wandlerelemente in Abhängigkeit von ihrer jeweiligen Lichtbeaufschlagung ein der Lichtstärke entsprechendes elektrisches Signal generieren, wobei der Ausgang eines jeden Abbildungssystems (5 - 7; A - G) zum Abbilden der von diesem Abbildungssystem bereitgestellten optischen Information über einer diesem Abbildungssystem (5 - 7; A - G) zugeordneten, aus einem oder mehreren Wandlerelementen bestehende Wandlerelementgruppe (PC 5 - PC 13; PC_{A} - PC_{G}) angeordnet ist, sowie mit einer durch die elektrischen Ausgangssignale des Sensorarrays (2, 10) beaufschlagten Auswerteeinheit zum Ansteuern von Aktoren in Abhängigkeit von dem Ergebnis einer Auswertung der Objekt bezogenen Informationen, welche Abbildungssysteme im Bereich ihrer Ausgänge zu einer gegenständlichen Einheit zusammengefaßt und auf einem dem Sensorarray (2, 10) zugeordneten Schaltungsträger fixiert sind.

2. Einrichtung nach Anspruch 1, bei der sich die in jeder Wandlerelementgruppe (PC 5 - PC 13; PC_{A} - PC_{G}) zusammenfaßten Wandlerelemente von denjenigen der in den übrigen übrigen Wandlerelementgruppen (PC 5 - PC 13; PC_{A} - PC_{G}) zusammengefaßten Wandlerelemente unterscheiden.

3. Einrichtung nach Anspruch 1 oder 2, bei der zumindest eines der Abbildungssysteme (5 - 7; A - G) einen bildabbildenden Optikkörper, etwa eine Linse (5, E - F) aufweist und andere als optische Lichtfilter (6, 7, A - D) ausgebildet sind.

4. Einrichtung nach Anspruch 3, bei welcher der Optikkörper (5) zur Abbildung des Innenraums zur Durchführung einer Innenraumüberwachung des Kraftfahrzeugs und die Lichtleiter (6, 7) zur Durchführung etwa einer Regensensorik, einer Sonnenstandssensorik oder einer Fahrlichtsteuerung vorgesehen sind.

## Claims (Claims for the following Contracting State(s): DE, ES, FR, IT)

1. Opto-electronic monitoring apparatus for an automotive vehicle, having a plurality of optical imaging systems (5 - 7; A - G), which are associated with different objects to be monitored and, on the output side, act upon the photosensitive surface of a common photoelectrical sensor array (2, 10), which comprises a plurality of photoelectrical transducer elements, forming individual image points, in a two-dimensional arrangement, which transducer elements generate an electrical signal, corresponding to the light intensity, in dependence on their respective light impingement, the output of each imaging system (5 - 7; A - G) being disposed above a group of transducer elements (PC 5 - PC 13; PC_{A}- PC_{G}), which is associated with this imaging system (5 - 7; A - G) and comprises one or more transducer elements, to display the optical information provided by this imaging system, as well as an evaluation unit, which is acted upon by the electrical output signals of the sensor array (2, 10), to trigger actuators in dependence on the result of an evaluation of the object-related information, which imaging systems are combined to form one individual unit in the region of their outputs and are secured on a circuit carrier associated with the sensor array (2, 10).

2. Apparatus according to claim 1, wherein the individual transducer elements of the sensor array (2, 10) are freely addressable and readable.

3. Apparatus according to claim 1 or 2, wherein the transducer elements, combined in each group of transducer elements (PC 5 - PC 13; PC_{A} - PC_{G}), are distinguished from those transducer elements combined in the other groups of transducer elements (PC 5 - PC 13; PC_{A} - PC_{G}).

4. Apparatus according to one of claims 1 to 3, wherein at least one of the imaging systems (5 - 7; A - G) has an image-displaying optical body, for example a lens (5, E - F), and other systems are configured as optical photoconductors (6, 7, A - D).

5. Apparatus according to claim 4, wherein the optical body (5) for displaying the interior is provided to effect a monitoring of the interior of the automotive vehicle, and the photoconductors (6, 7) are provided, for example, to sense rain, to sense the brightness of the sun or to control headlights.

## Claims (Claims for the following Contracting State(s): GB)

1. Opto-electronic monitoring apparatus for an automotive vehicle, having a plurality of optical imaging systems (5 - 7; A - G), which are associated with different objects to be monitored and, on the output side, act upon the photosensitive surface of a common photoelectrical sensor array (2, 10), which comprises a plurality of photoelectrical, freely addressable and readable transducer elements, forming individual image points, in a two-dimensional arrangement, which transducer elements generate an electrical signal, corresponding to the light intensity, in dependence on their respective light impingement, the output of each imaging system (5 - 7; A - G) being disposed above a group of transducer elements (PC 5 - PC 13; PC_{A} - PC_{G}), which is associated with this imaging system (5 - 7; A - G) and comprises one or more transducer elements, to display the optical information provided by this imaging system, as well as an evaluation unit, which is acted upon by the electrical output signals of the sensor array (2, 10), to trigger actuators in dependence on the result of an evaluation of the object-related information, which imaging systems are combined to form one individual unit in the region of their outputs and are secured on a circuit carrier associated with the sensor array (2, 10).

2. Apparatus according to claim 1, **characterised in that** the transducer elements, combined in each group of transducer elements (PC 5 - PC 13; PC_{A} - PC_{G}), are distinguished from those transducer elements combined in the other groups of transducer elements (PC 5 - PC 13; PC_{A} - PC_{G}).

3. Apparatus according to claim 1 or 2, **characterised in that** at least one of the imaging systems (5 - 7; A - G) has an image-displaying optical body, for example a lens (5, E - F), and other systems are configured as optical photoconductors (6, 7, A - D).

4. Apparatus according to one of claims 1 to 3, **characterised in that** the optical body (5) for displaying the interior is provided to effect a monitoring of the interior of the automotive vehicle, and the photoconductors (6, 7) are provided, for example, to sense rain, to sense the brightness of the sun or to control headlights.

## Revendications (Revendications pour l'(les) Etat(s) contractant(s) suivant(s): DE, ES, FR, IT)

1. Système de surveillance optoélectronique pour automobile, avec plusieurs systèmes de représentation optique (5 - 7 ; A - G) affectés à divers objets à surveiller, qui transmettent, du côté sortie, des signaux sur la surface photosensible d'un détecteur photoélectrique en ligne (2, 10) commun, constitué d'une multitude d'éléments capteurs photoélectriques, formés par des points d'image, en disposition bidimensionnelle, lesquels éléments capteurs génèrent, en fonction des signaux lumineux qui leur sont transmis, un signal électrique correspondant à une intensité lumineuse, sachant qu'à la sortie de chaque système de représentation (5 - 7 ; A - G) est disposé, à des fins de représentation des informations optiques mises à disposition par le système de représentation, un groupe d'élément capteur (PC 5 - PC 13 ; PC_{A} - PC_{G}) constitué d'un ou de plusieurs éléments capteurs, ainsi qu'avec une unité d'exploitation recevant les signaux électriques à la sortie du détecteur en ligne (2, 10) en vue de piloter des acteurs en fonction du résultat de l'exploitation des informations se rapportant à l'objet, lesquels systèmes de représentation sont regroupés au niveau de leurs sorties en une unité par objet et fixés sur un support de connexion affecté au détecteur en ligne (2, 10).

2. Système selon la revendication 1 pour lequel les différents éléments capteurs du détecteur en ligne (2, 10) peuvent être adressés et sélectionnés librement.

3. Système selon la revendication 1 ou 2 pour lequel les éléments capteurs regroupés dans chaque groupe d'éléments capteurs (PC 5 - PC 13 ; PC_{A}- PC_{G}) se différencient des autres éléments capteurs regroupés dans les autres groupes d'éléments capteurs (PC 5 - PC 13; PC_{A}-PC_{G})

4. Système selon l'une des revendications 1 à 3 pour lequel au moins l'un des systèmes de représentation (5 - 7 ; A - G) présente un corps optique de représentation, par exemple une lentille (5, E - F) et que les autres soient conçus en tant que câble de fibres optiques (6, 7, A - D).

5. Système selon la revendication 4, pour lequel le corps optique (5) de représentation de l'habitacle est prévu pour surveiller l'habitacle d'une automobile et les câbles de fibres optiques (6, 7) sont prévus dans le cadre d'un système de détection de pluie, de détection d'ensoleillement ou de commande des feux.

## Revendications (Revendications pour l'(les) Etat(s) contractant(s) suivant(s): GB)

1. Système de surveillance optoélectronique pour automobile, avec plusieurs systèmes de représentation optique (5 - 7 ; A - G) affectés à divers objets à surveiller, qui transmettent, du côté sortie, des signaux sur la surface photosensible d'un détecteur photoélectrique en ligne (2, 10) commun, constitué d'une multitude d'éléments capteurs photoélectriques, formés par des points d'image, librement adressables et sélectionnables, en disposition bidimensionnelle, lesquels éléments capteurs génèrent, en fonction des signaux lumineux qui leur sont transmis, un signal électrique correspondant à une intensité lumineuse, sachant qu'à la sortie de chaque système de représentation (5 - 7 ; A - G) est disposé, à des fins de représentation des informations optiques mises à disposition par le système de représentation, un groupe d'élément capteur (PC 5 - PC 13 ; PC_{A} - PC_{G}) constitué d'un ou de plusieurs éléments capteurs, ainsi qu'avec une unité d'exploitation recevant les signaux électriques à la sortie du détecteur en ligne (2, 10) en vue de piloter des acteurs en fonction du résultat de l'exploitation des informations se rapportant à l'objet, lesquels systèmes de représentation sont regroupés au niveau de leurs sorties en une unité par objet et fixés sur un support de connexion affecté au détecteur en ligne (2, 10).

2. Système selon la revendication 1 pour lequel les éléments capteurs regroupés dans chaque groupe d'éléments capteurs (PC 5 - PC 13 ; PC_{A} - PC_{G}) se différencient des autres éléments capteurs regroupés dans les autres groupes d'éléments capteurs (PC 5 - PC 13 ; PC_{A} - PC_{G}).

3. Système selon la revendication 1 ou 2 pour lequel au moins l'un des systèmes de représentation (5 - 7 ; A - G) présente un corps optique de représentation, par exemple une lentille (5, E - F) et que les autres soient conçus en tant que câble de fibres optiques (6, 7, A - D).

4. Système selon l'une des revendications 1 à 3 pour lequel le corps optique (5) de représentation de l'habitacle est prévu pour surveiller l'habitacle d'une automobile et les câbles de fibres optiques (6, 7) sont prévus dans le cadre d'un système de détection de pluie, de détection d'ensoleillement ou de commande des feux.
